(19)

Europäisches
Patentamt

European
Patent Office

Office européen
des brevets

(11)  **EP 3 099 807 B1**

(12)  **EUROPEAN PATENT SPECIFICATION**

(45) Date of publication and mention
of the grant of the patent:
**13.02.2019 Bulletin 2019/07**

(51) Int Cl.:
**C12P 21/00** (2006.01)    **G06F 19/00** (2018.01)

(21) Application number: **14830805.9**

(86) International application number:
**PCT/EP2014/078340**

(22) Date of filing: **17.12.2014**

(87) International publication number:
**WO 2015/113704 (06.08.2015 Gazette 2015/31)**

(54) **A METHOD OF PREDICTING RELATIVE FED BATCH PRODUCTION TITER OF A PANEL OF CLONALLY-DERIVED PRODUCER CELLS**

VERFAHREN ZUR VORHERSAGE EINES RELATIVEN FED-BATCH-HERSTELLUNGSTITERS EINER PLATTE AUS KLONAL ABGELEITETEN HERSTELLERZELLEN

PROCÉDÉ DE PRÉDICTION DE TITRE DE PRODUCTION EN CUVÉE-ALIMENTÉE RELATIVE D'UN PANNEAU DE CELLULES PRODUCTRICES ISSUES DE CLONAGE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.01.2014 EP 14153333**

(43) Date of publication of application:
**07.12.2016 Bulletin 2016/49**

(73) Proprietor: **Valitacell Limited**
**Co. Tipperary Ballina (IE)**

(72) Inventors:
• **THOMPSON, Ben**
**Sheffield**
**Yorkshire S11 8YJ (GB)**
• **JAMES, David**
**Sheffield**
**Yorkshire S11 9LN (GB)**
• **CLIFFORD, Jerry**
**Co. Kerry (IE)**

(74) Representative: **Carmody, Mark**
**PurdyLucey**
**Intellectual Property Limited**
**6-7 Harcourt Terrace**
**D02 FH73 Dublin 2 (IE)**

(56) References cited:
• **RACHEL LEGMANN ET AL: "A Strategy for clone selection under different production conditions", BIOTECHNOLOGY PROGRESS, vol. 27, no. 3, 29 May 2011 (2011-05-29), pages 757-765, XP055084189, ISSN: 8756-7938, DOI: 10.1002/btpr.577**
• **JIANLIN XU ET AL: "Galactose can be an inducer for production of therapeutic proteins by auto-induction usingBL21 strains", PROTEIN EXPRESSION AND PURIFICATION, vol. 83, no. 1, 18 June 2005 (2005-06-18), pages 30-36, XP028413544, ISSN: 1046-5928, DOI: 10.1016/J.PEP.2012.02.014 [retrieved on 2012-03-08]**
• **HAIMANTI DORAI ET AL: "Combining high-throughput screening of caspase activity with anti-apoptosis genes for development of robust CHO production cell lines", BIOTECHNOLOGY PROGRESS, vol. 26, no. 5, 24 September 2010 (2010-09-24), pages 1367-1381, XP055123365, ISSN: 8756-7938, DOI: 10.1002/btpr.426**
• **DA CRUZ MELEIRO L A ET AL: "Non-Linear Multivariable Predictive Control of an Alcoholic Fermentation Process Using Functional Link Networks", BRAZILIAN ARCHIVES OF BIOLOGY AND TECHNOLOGY, INSTITUTO DE TECNOLOGIA DO PARANA, BR, vol. 48, no. Special, 1 June 2005 (2005-06-01), pages 7-18, XP002490882, ISSN: 1516-8913**

EP 3 099 807 B1

**Description**

Introduction

The invention relates to a method of predicting fed batch production titer of a producer cell. In particular, the invention relates to a method of predicting relative fed batch recombinant protein titer of a panel of clonally derived producer cells.

Background to the Invention

Early prediction of the relative ability of CHO cell clonal isolates to serve as manufacturing cell lines is a key component of the cell line development process. Clones apparently productive in static culture can vary substantially in subsequent fed-batch culture performance, both with respect to cell growth and productivity. Current best practice is to measure clone performance early in cell line development using a small-scale, multi-parallel bioreactor system that enables comparison of 50-100 clonal isolates. However, this approach is still both costly and time-consuming.
It is an object of the invention to overcome at least one of the above-referenced problems.

Statements of Invention

The Applicant has discovered that the production titer of producer cell clones in fed batch culture can be accurately predicted by rapid profiling of clone-specific recombinant protein production response to one or more functionally diverse chemical stressors (for example inhibitors and toxins) in static microplate culture. To perform microplate profiling, a fixed number of clonally-derived cells were dispensed into 96-well microplates containing one or more chemical cell stressors separately loaded into discrete wells. Each chemical was soluble in cell growth medium and typically utilized at a concentration pre-determined to inhibit parental CHO cell proliferation in microplates within the $LD_{30}$ to $LD_{80}$ range. After static growth for a growth period (i.e.3 days), supernatant MAb titer was measured by HPLC Protein A assay. Twelve CHO-S clones expressing an IgG1 MAb isolated by limiting dilution cloning were subjected to microplate profiling followed by assessment of optimized fed-batch culture performance (MAb titer [MAb], integral of viable cell concentration to 50% cell viability [$IVCC_{50}$]) in Ehrlenmayer flasks. Using multi-linear modeling, it was demonstrated that clone performance in microplates could predict MAb titer ($r^2 = 0.84$) using clone-specific MAb titer microplate profiles.
The invention is set out in the appended claims.
Described herein is a computer-implemented method of predicting production titer of a query producer cell in fed batch culture, the method comprising the steps of:

- incubating the query producer cell with one or more chemical cell stressors;
- determining the production titer response of the query producer cell in the presence of the or each chemical cell stressor to generate a query cell-specific production titer response profile;
- inputting the query cell-specific production titer response profile into a computational model, in which the computational model is generated from production titer response profiles obtained from a calibration set of cells with known fed batch production titer, wherein the computational model is configured to output the predicted fed batch production titer for the cell.

An aspect of the invention provides a rapid, high-throughput, computer-implemented method of predicting relative production titer of a panel of clonal producer cells in fed batch culture, the method comprising the steps of:

- simultaneously incubating each producer cell with at least three individual chemical cell stressors in a static microplate culture for an incubation period of less than four days, in which each producer cell is incubated with a single chemical cell stressor provided at a concentration of 0.5 to 2.0 ICso;
- determining the production titer response of each cell in the presence and absence of the at least three chemical cell stressors to generate a cell-specific production titer response profile for each of the panel of producer cells;
- inputting the cell-specific production titer response profile for each of the panel of producer cells into a computational model, in which the computational model is generated from production titer response profiles obtained from a calibration set of producer cells with known fed batch production titer, wherein the computational model is configured to output the predicted relative fed batch production titer of the panel of producer cells.

When a batch of new clones are generated from a parental cell line, rather than all or a select few being subjected to expensive and time-consuming growth studies in bioreactors, the method of the invention allows a large number of clones to be rapidly assayed for, and ranked according to, predicted fed batch (recombinant protein) production titer in a rapid and high-throughput manner. This informs on which clones to take forward to scale-up/mini-bioreactor studies, for example only the good clones as illustrated in Fig. X below.

The methods of the invention employs data (production titer response profile) from a panel of pre-validated clones (clones of known production titer), and a computational model, ideally a multiple linear computational model, based on this data, to allow prediction of relative fed batch recombinant production titer of a new set of clones based on their rapidly obtained chemical fingerprints.

The method comprises an aditional step of ranking the clones according to predicted fed batch production titer.

The method is typically carried out using a microtiter plate, in which each assay is performed in a well of a microtiter plate. Suitably, the growth of each clonal cell is assayed in the well of a microtiter plate. Multiple chemical cell stressors are employed and each clone will be assayed in the presence of single chemical cell stressor.

The assay involves mixing a sample of a clone with a chemical cell stressor, and incubating the mixture from 1 to 4 days, typically 2 to 4 days, preferably 60-80 hours, and ideally about 3 days, and assaying the level of growth of the cells. Suitably, the clone sample is provided at a concentration of from 0.1 to 1.0 x $10^6$ cells per ml of mixture. The chemical cell stressor is provided at a concentration of 0.5 to 2 x IC50, ideally about 1 x IC50.

Suitably, the growth of each clone is assayed after a period of incubation of less than 5 days. Ideally, the growth of each clone is assayed after a period of incubation of between 1-4 days, and ideally after 2 and 3 days.

Preferably, the growth of each clone is assayed simultaneously. The incubation step is carried out in static microplate culture.

In an embodiment, the invention provides a rapid, high-throughput, computer-implemented method of predicting relative production (i.e. monoclonal antibody) titer of a panel of clonal producer cells in fed batch culture, the method comprising the steps of:

- simultaneously incubating each producer cell with at least three individual, functionally diverse, chemical cell stressors in static microplate culture for an incubation period of less than four days, in which each producer cell is incubated with a single chemical cell stressor provided at a concentration of 0.5 to 2.0 IC50;
- after the incubation period determining the production titer response of each cell in the presence of each individual chemical cell stressors to generate a cell-specific production titer response profile for each of the panel of producer cells;
- inputting the cell-specific production titer response profile for each of the panel of producer cells into a computational model, in which the computational model is generated from production titer response profiles obtained from a calibration set of producer cells with known fed batch production titer, wherein the computational model is configured to output the predicted relative fed batch production titer of the panel of producer cells.

The chemical cell stressors are chemicals that cause a reduction in cell growth via one or more of multiple cellular pathways. Suitably, the plurality of chemical cell stressors comprise at least 2, 3, 4, 5, or 6 functionally diverse chemical cell; stressors, for example at least 3, 4, 5, 6, 7, 8, 9, 10, 12, 14, 16, 18, 20, 21, 22, 23, 24, or 25 different stressors. The plurality of chemical cell stressors are selected from the group consisting of amino acid transport inhibitors, cell cycle inhibitors, a source of osmotic stress, a source of oxidative stress, an inducer of apoptosis, metabolic effectors, a pH modifier, an inhibitor of glycolysis, and a toxin. These are examples of functionally diverse chemical cell stressors. Typically, the plurality of chemical cell stressors include stressors selected from at least 4, 5, 6 or 7 of the groups consisting of amino acid transport inhibitors, cell cycle inhibitors, a source of osmotic stress, a source of oxidative stress, an inducer of apoptosis, metabolic effectors, a pH modifier, an inhibitor of glycolysis, and a toxin.

Described herein is a microtiter plate comprising at least 24, 48 or 96 wells, and a plurality of chemical cell stressors disposed individually in at least some of the wells. Typically, the plate comprises at least 6, 7, 8, 9, 10, 12, 14, 16, 18, 20, 21, 22, 23, 24, or 25 chemical cell stressors. Suitably, each chemical cell stressor is disposed in at least two or three wells of the plate (i.e. duplicate or triplicate).

Preferably, the plurality of chemical cell stressors are selected from the group of 2-aminobicyclo-(2,2,1)heptane-carboxylic acid (BCH), D-phenylalanine, $\alpha$-(methylamino)isobutyric acid (MeAIB), Sodium Butyrate (NaBu), cycloheximide, ammonium chloride, Cadmium acetate dihydrate, Cobalt chloride (CoC12), Sodium Chloride (NaCl), Sodium lactate (Na Lac), Aminotriazole (AMT), Menadione Sodium Bisulphite (MSB), Buthionine Sulfoximine (BSO), Mercaptosuccinic Acid (MS), 2,4,Dinitrophenol (24DNP), Sodium Oxamate, 2-deoxyglucose (2dg), 3-bromopyruvate (3-BrPA), Dichloroacetate (DCA), 6-diazo-5-oxo-1-norleucine (1-don), Valproic acid (Val), Sodium Orthovandate (NaV), citric acid, FK866, lactic acid.

Described herein is a microtiter plate comprising at least 24, 48 or 96 wells, and one or more, generally a plurality of, chemical cell stressors disposed individually in at least some of the wells, in which when a plurality of different chemical cell stressors is employed the chemical cell stressors comprise at least one chemical cell stressor selected from each of the groups consisting of amino acid transport inhibitors, cell cycle inhibitors, a source of osmotic stress, a source of oxidative stress, an inducer of apoptosis, metabolic effectors, a pH modifier, an inhibitor of glycolysis, and a toxin.

Described herein is a microtiter plate comprising at least 96 wells, and at least 23 chemical cell stressors disposed individually in wells of the plate, in which the chemical cell stressors consist essentially of 2-aminobicyclo-(2,2,1)heptane-carboxylic acid (BCH), D-phenylalanine, α-(methylamino)isobutyric acid (MeAIB), Sodium Butyrate (NaBu), cycloheximide, ammonium chloride, Cadmium acetate dihydrate, Cobalt chloride (CoCl2), Sodium Chloride (NaCl), Sodium lactate (Na Lac), Aminotriazole (AMT), Menadione Sodium Bisulphite (MSB), Buthionine Sulfoximine (BSO), Mercaptosuccinic Acid (MS), 2,4,Dinitrophenol (24DNP), Sodium Oxamate, 2-deoxyglucose (2dg), 3-bromopyruvate (3-BrPA), Dichloroacetate (DCA), 6-diazo-5-oxo-1-norleucine (1-don), Valproic acid (Val), Sodium Orthovandate (NaV), citric acid, FK866, lactic acid.

Described herein is a kit suitable for performing a method described herein and comprising (a) a microtiter plate described above (b) a microtiter plate reader, and (c) a computer program comprising program instructions for (i) receiving from a determination system (for example a HPLC) a production titer value of each clone in the presence and absence of the or each chemical cell stressor, (ii) calculating a normalised production titer value for each clone in the presence of the or each chemical cell stressor, (ii) inputting a clone-specific production titer response profile comprising the normalised production titer value for each clone in the presence of the or each chemical cell stressor into a computational model, in which the computational model is generated from production titer response profiles obtained from a calibration set of clones with known production titer values, wherein the computational model is configured to output the predicted fed batch production titer value for each clone (and optionally predict the relative fed batch production titer value of the panel of clonal cells), and (iii) outputting the predicted fed batch production titer value for each clone (and optionally predict the relative fed batch production titer value of the panel of clonal cells),

Described herein is a kit suitable for performing a method of predicting the relative fed batch production titer of a panel of clonal producer cells derived from a single parental host cell population and comprising (a) a microtiter plate described above (b) a microtiter plate reader, and (c) a computer program comprising program instructions for (i) receiving from a suitable machine a production titer value of each clone grown in the presence and absence of one or more chemical cell stressors, (ii) calculating a normalised production titer value for each clone in the presence of the or each chemical cell stressor, (ii) inputting a clone-specific production titer response profile comprising the normalised production titer value for each clone in the presence of the or each chemical cell stressor into a computational model, in which the computational model is generated from production titer response profiles obtained from a calibration set of clones with known production titer values, wherein the computational model is configured to output the predicted fed batch production titer value for each clone and predict the relative fed batch production titer value of the panel of clonal cells, and (iii) outputting the predicted relative fed batch production titer value of the panel of clonal cells.

The determination system employed to determine the production titer of a clone may be any suitable machine, for example a HPLC or mass spectrometer adapted to quantitatively assay the target recombinant protein.

Described herein is a computer implemented system for performing a method of predicting fed batch production titer of a clonal producer cell derived from a single cell line, the system comprising:

- a determination system for assaying production titer of the clone in the presence and absence of one or more individual chemical cell stressors (typically a plurality of individual chemical cell stressors) to provide a normalised production titer response value for the clone in one or more stressed microenvironments;
- a computational model adapted to process a clone-specific production titer response profile comprising the normalised production titer response value for the clone in the or each stressed microenvironment, in which the computational model is generated from production titer response profiles obtained from a calibration set of clones with known fed batch production titer response profiles, wherein the computational model is configured to output the predicted fed batch production titer value for the clone, and
- means for outputting the predicted fed batch production titer for the clonal producer cell.

Also described herein is a computer implemented system for performing a method of predicting relative fed batch production titer of a panel of clonal producer cells derived from a single cell line, the system comprising:

- a determination system for assaying production titer of each clone in the presence and absence of a plurality of individual chemical cell stressors to provide a normalised production titer response value for each clone in a plurality of stressed microenvironments;
- a computational model adapted to process a clone-specific production titer response profile comprising the normalised production titer response value for each clone in each of the stressed microenvironments, in which the computational model is generated from production titer response profiles obtained from a calibration set of clones with known fed batch production titer response profiles, wherein the computational model is configured to output the predicted fed batch production titer value for each clone and/or the predicted relative fed batch production titer of the panel of clonal cells, and
- means for outputting (i) the predicted fed batch production titer for one or more clones in the panel, or (ii) the predicted relative fed batch performance of the panel of clonal cells, or (iii) both (i) and (ii).

An aspect of the invention provides a computer-implemented method of predicting production titer of a query producer cell in fed batch culture, the method comprising the steps of:

- incubating the query producer cell with at least three chemical cell stressors individually in static microplate culture, each chemical cell stressor provided at a concentration of 0.5 to 2.0 IC50;
- determining the production titer response of the query producer cell in the presence of each chemical cell stressor to generate a query cell-specific production titer response profile;
- inputting the query cell-specific production titer response profile into a computational model, in which the computational model is generated from production titer response profiles obtained from a calibration set of cells with known fed batch production titer, wherein the computational model is configured to output the predicted fed batch production titer for the cell.

Typically, the determination system comprises a HPLC, although other systems adapted to quantitatively assay for a target protein, for example a quantitative ELISA, may be employed.

Suitably, the computational model is a multiple linear computational model. Ideally, the multiple linear computational model suitably employs a least squares solution to fit parameters (i.e. levels of cell specific production titer responses) to the observed fed batch production titer.

The invention also provides a computer program which when executed on a computer causes the computer to perform a method of predicting relative fed batch production titer of a panel of clonal cells derived from a single cell line according to the methods of the invention.

The invention also relates to a computer program recording medium storing a computer program according to the invention.

Brief Description of the Figures

**Figure 1:** Maximum monoclonal antibody titer achieved in fed batch culture from a panel of 12 clonal cell lines from a common parental cell line.

**Figure 2:** The range of product titers in the presence of specific chemical stressors achieved by a panel of 12 clonal cell lines.

**Figure 3:** A visual representation of the goodness of fit of a multiple linear model to predict maximum MAb titer using chemical specific MAb responses.

**Figure 4:** An illustration of the ability of the model to predict new data. Here, using the stated model parameters, Max MAb titer was predicted with new data using a "leave one out" cross validation function.

Detailed Description of the Invention

Described herein is a method of screening a panel of producer cell clones derived from a single cell line to stratify the clones according to fed batch production titer. The method involves incubating a sample of each clone with and without one, and generally a plurality of, individual chemical cell stressors for a period of time, typically of at least 24 hours and up to 4 or 5 days, to obtain a plurality of normalised production titer response values for the clone. These production titer response value(s) provide a specific pattern of clone-specific production titer response that forms a clone-specific growth response profile. A multiple linear computational model is employed to correlate the production titer response profiles with a plurality of production titer response profiles from a calibration set of clones with know fed batch production titer, and predict a fed batch production titer for one or more of the panel of clones. The clones from the panel may then be ranked according to predicted fed batch production titer, which allows producer clones to be chosen for further development.

In this specification, the term "rapid, high-throughput" should be understood to mean that the method can be carried out in four days or less, and in which the incubation of cells during the incubation period may be carried out in the wells of a microtiter plate.

The term "production titer" or "production titer response"as applied to a specific producer cell clone refers to the amount of a specific protein, generally a recombinant protein, and ideally a recombinant monoclonal antibody, that the specific producer cell clone generates over a defined time period. The titer may be quantified in absolute or relative terms. Generally titer is referred to as weight of product per volume of culture - grams per litre (g/L) is a common metric (Max titer). Generally, the clones are incubated for a specific time period, for example 2-4 days, and following the incubation period a sample of the supernatant is typically taken and assayed for production titer. Various methods will be apparent to the person skilled in the art for measuring production titer, including HPLC and quantitative ELISA.

In many cases, the production titer employed in the methods of the invention will be normalised production titer response, meaning the difference between the production titer response for the cell when measured in the presence and absence of the chemical cell stressor.

The term "producer cell" refers to a cell that is employed to generate a specific desired protein. Generally, the cell is genetically modifed to include one or multiple copies of a transgene encoding the desired protein which is generally under the control of a specific promotor. Thus, the specific protein is usually a recombinant protein. Producer cells are well known in the art, and include for example Chinese hamster ovary (CHO) cells or baby hamster kidney (BHK) cells. Ideally, the producer cell is a CHO cell. Typically, the producer cell is a monoclonal antibody producer cell.

The term "production titer response profile" refers to the production titer responses for a given clone to one or more chemical cell stressors. In one embodiment, the profile may include a single production titer response for a given clone (the production titer response for the cell in the presence and typically absence of one chemical cell stressor). In other embodiments, the production titer response profile comprises a plurality of production titer responses or a given clone (the production titer responses for the cell in the presence, and optionally absence) of a plurality of individual chemical cell stressor). Generally, the profile is generated using normalized production titer responses (production titer in absence of stressor minus production titer in presence of stressor), so that a normalized production titer response profile is obtained. However, in circumstances where the profile is generated using more than one stressor, it is possible to use profiles generated with production titers from stressed microenvironments only (i.e. without control production titers).

The method of the invention employs rapid profiling of producer cells to predict the production titer of the cells when in fed batch culture. In this specification, the term "fed batch" or "fed batch culture" should be understood to mean extended culture of cells where additional nutrients are added in bolus format at least once post seeding of the cells.

The term "calibration set of clones" refers to a plurality of clones, each having a production titer response fingerprint and a known fed batch performance. Typically, the calibration set of clones includes clones exhibiting a range of fed batch performance abilities from what the end user would consider 'good' performers to what the end user would consider 'bad' performers. Suitably, the calibration set of clones comprises at least 3, 4, 5, 6, 7, 8, 9, 10, 12, 14, 16, 18, 20, 22, 24, or 26 clones

The term "stressed microenvironment" should be understood to mean an environment that includes a chemical cell stressor. Generally, this means that a clone is assayed for growth in a well of microtiter plate in the presence of the chemical cell stressor.

In this specification, the term "rapid" should be understood to mean a method of predicting fed batch performance that takes less than five days, and ideally less than four or three days.

In this specification, the term "high-throughput" should be understood to mean a method in which a large number of samples, for example 20-500, can be assayed simultaneously. In one embodiment, this involves assaying the growth of the clones in a multiwell plate, for example a 48, 96, or 192 well plate.

In this specification, the term "predicted fed batch production titer" as applied to a clonal producer cell should be understood to mean the predicted fed batch production titer of the clone in fed batch culture. The predicted production titer may be quantified in absolute terms, or may be quantified in relative terms, i.e. relative to a clone having known good or bad production titer or relative to a panel of clones.

In this specification, the term "predicting relative production titer" should be understood to mean predicting fed batch production titer of the clones in the panel relative to one another. Thus, in one embodiment, the clones are stratified according to their predicted fed batch production titer. Likewise, the term "predicted relative fed batch production titer of a panel of clonal cells" should be understood to mean the predicted fed batch production titer of the clones in the panel relative to one another. Thus, in one embodiment, the clones are stratified according to their predicted fed batch production titer. In another embodiment, the clones are stratified to pick one or more clones showing best predicted fed bacth production titer clones, one or more clones showing worst predicted fed bacth production titer clones, or both.

In this specification, the term "panel of clonal producer cells" should be understood to mean a panel of clonal producer cell populations derived from a single cell line, and comprising from 2 to 500 or more clonal producer cell populations. Methods for generating panels of clonal producer cells are well known to a person skilled in the art, and described in Production of recombinant protein therapeutics in cultivated mammalian cells (2004),Wurm, Florian M, New York, NY, Nature Biotechnology 22 (2004), S. 1393-1398. Typically, the panel of clonal producer cell populations include from 10-500, 20-500, 30-500, 40-500, 50-500, 60-500, 70-500, 80-500, 90-500 or 100-500 clonal cell populations. Typically, the panel of clonal producer cell populations include from 100-500, 100-400, 150-400, 150-350 clonal cell populations.

In this specification, the term "computational model" typically refers to a multiple linear computational model. Ideally, the multiple linear computational model employs a least squares solution to fit parameters (i.e. levels of cell specific production titer responses) to the observed fed batch production titer.

In this specification, the term "chemical cell stressor" should be understood to mean a chemical that can be incubated with a cell in a cell culture medium and is capable of causing a reduction in cell growth via one or more cellular pathways. Preferably, the chemical cell stressor is soluble in cell culture medium. Examples of chemical cell stressors (cell stressor types) include inhibitors of cellular pathways, cell toxins (chemicals that are toxic to cells, especially mammalian cells), metabolic effectors, and chemicals that stress the cells. Examples of inhibitors include amino acid transport inhibitors, cell cycle inhibitors, and glycolysis inhibitors. Examples of chemicals that stress cells are sources of osmotic or oxidative stress. Typically, the chemical cell stressor is utilised in an amount that inhibits cell proliferation within the $LD_{30}$ to $LD_{80}$ range. Suitably, the chemical cell stressor is utilised at a concentration of 0.5 to 2 x $IC_{50}$. Typically, a sample of each cell (i.e., a sample of each clone) is separately incubated with at least three different cell stressors. Preferably, a sample of each cell (i.e., a sample of each clone) is separately incubated with at least three different types of cell stressors (for example, an amino acid transport inhibitor, a glycolysis inhibitor, and a source of osmotic stress). Suitably, the plurality of chemical cell stressors comprise at least 3, 4, 5, 6, 7, 8, 9, 10, 12, 14, 16, 18, 20, 21, 22, 23, 24, or 25 stressors. Typically, the plurality of chemical cell stressors are selected from the group consisting of amino acid transport inhibitors, cell cycle inhibitors, a source of osmotic stress, a source of oxidative stress, an inducer of apoptosis, metabolic effectors, a pH modifier, an inhibitor of glycolysis, and a toxin. Typically, the plurality of chemical cell stressors include stressors selected from at least 4, 5, 6 or 7 of the groups consisting of amino acid transport inhibitors, cell cycle inhibitors, a source of osmotic stress, a pH modifier, a source of oxidative stress, an inducer of apoptosis, metabolic effectors, an inhibitor of glycolysis, and a toxin. Examples of chemical cell stressors include:

    BCH (AA transport Inhibitor)
    D-phenylalanine (AA transport Inhibitor)
    MeAIB (AA transport inhibitor)
    Sodium Butyrate (Cell cycle inhibitor)
    Cycloheximide (Toxin)
    Ammonium chloride (Toxin)
    Cobalt chloride (inducer of apoptosis)
    NaCL (source of osmotic stress)
    Sodium lactate (Metabolic effector)
    Aminotraizole (source of oxidative stress)
    Menadione sodium bisulphate (source of oxidative stress)
    Mercaptosuccinic acid (source of oxidative stress)
    2, 4 dinitrophenol (Metabolic effector)
    Sodium oxamate (Glycolysis inhibitor)
    2 deoxyglucose (Glycolysis inhibitor)
    3 bromopyruvate (Glycolysis inhibitor)
    Dichloroacetate (Glycolysis inhibitor)
    L-don (AA synthesis inhibitor)
    Valproic acid (cell cycle inhibitor)
    sodium orthovandate (cell cycle inhibitor)
    FK866 (apoptosis inducer)
    citrate/lactate (pH modifier)

"IC50": To establish inhibitory concentrations, "in house parental CHO" cells were incubated in 96 well plates in the presence of a range of concentrations of the chemical of interest for three days (72 hours). Growth was determined using a chemical viable cell number assay("Presto Blue" from Life Technologies, UK). Growth in the presence of the chemical concentrations was compared to the control cell growth (i.e. in the presence of 0mM inhibitory chemical) to produce a curve of chemical concentration vs percentage growth relative to the control. This curve was used to establish IC "X%" concentrations.

Exprimental

*Cell culture:*

Clones used were CHO-S derived clones from a monoclonal antibody producing parental clone (clone 38) provided by Cobra biologics (Keele, UK). These are henceforth referred to as "PM-CHO". PM-CHO Cells were cultured in CD-CHO media (Invitrogen, Paisley, UK), 8mM L-Glutamine (Invitrogen, Paisley, UK), 1% HT supplement (Invitrogen, Paisley, UK) 12.5 µg/ml puromycin (Invitrogen, Paisley, UK). Cells were routinely sub-cultured on an alternating 3 / 4 day regime.

*Fed batch studies:*

Fed batch studies were performed in 60 ml volumes in shake flasks using commercially available feed and media. Feed was added in 6 ml boluses on days 4,6,8 and 10. Samples were taken daily and analysed for cell growth and monoclonal antibody titer.

*Determination of Monoclonal antibody titer:*

A HPLC method was employed using a "Biomonolith" protein A column (Aglient, Workingham, UK). Monoclonal antibody was quantified according to area under UV absorption peak in the eluent. This was demonstrated to be linearly proportional to antibody concentration in the sample.

*Method of performing invention:*

From a detailed literature survey, a variety of chemicals which were anticipated to be informative of fed batch performance were identified. These were:

2-aminobicyclo-(2,2,1)heptane-carboxylic acid (BCH)
D-phenylalanine -(D-Phe)
$\alpha$-(methylamino)isobutyric acid (MeAIB)
Sodium Butyrate (NaBu)
Cycloheximide
Ammonium chloride
Cadmium acetate dihydrate
Cobalt chloride (CoC12)
Sodium Chloride (NaCl)
Sodium lactate (Na Lac)
Aminotriazole (AMT)
Menadione Sodium Bisulphite (MSB)
Buthionine Sulfoximine (BSO)
Mercaptosuccinic Acid (MS)
2,4,Dinitrophenol (24DNP)
Sodium Oxamate
2-deoxyglucose (2dg)
3-bromopyruvate (3-BrPA)
Dichloroacetate (DCA)
6-diazo-5-oxo-1-norleucine (1-don)
Valproic acid (Val)
Sodium Orthovandate (NaV)
Citric acid
FK866
Lactic acid

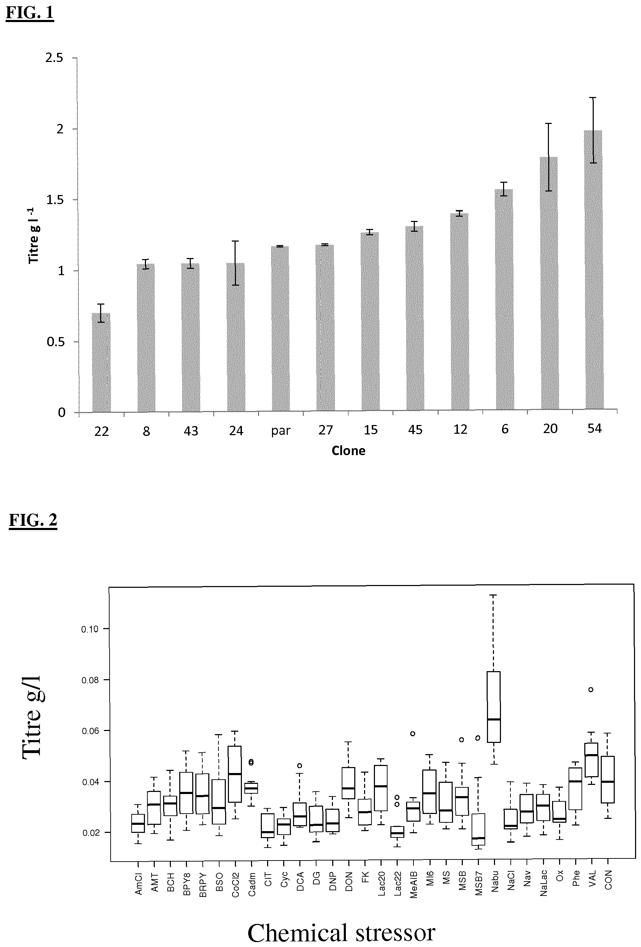

Using an 'in house' parental cell line, inhibitory dose 50 ($IC_{50}$) concentrations of the above chemicals were identified through growth studies. Here $IC_{50}$ is defined as the concentration of the chemical, in question, which inhibits normal cell growth by 50% over a 3 day period. Once $IC_{50}$s were established, 96 well plates were set up containing the above selection of chemicals at the $IC_{50}$ concentration and including control wells which contained only cell growth media.
A panel size of 12 of monoclonal antibody producing clonal cell lines was generated in house by a limiting dilution cloning method using the PM parental cell line. These clones were subjected to fed batch studies to assess their fed batch product production ability. Here this is defined as the maximum volumetric product titer achieved during the fed batch run. The range of maximum titers achieved during fed batch by the clonal panel is illustrated in figure 1.
Each clonal cell line was grown in a 96 well plate containing the above mentioned chemicals for 3 days. After this period the level of chemical specific product titer in the plates (i.e. MAb titer in the presence and absence of chemicals as mentioned above - normalized production titer) was measured using the "HPLC" assay as described above. This allowed a chemical specific product production fingerprint to be identified for each clonal cell line (referred to above as a clone specific production titer response profile). Here a product production fingerprint is defined as the plurality of product production levels in each chemical microenvironment.

The range of chemical specific productivities for the panel of clones is illustrated in Fig. 2.

Using information from these chemical specific product production fingerprints, a multiple linear model was built using responses to individual chemicals as parameters.

A multiple linear model is defined as that satisfying the equation.

$$\hat{\beta} = (\mathbf{X}^{\mathrm{T}}\mathbf{X})^{-1}\mathbf{X}^{\mathrm{T}}\mathbf{y} = \left(\tfrac{1}{n}\sum \mathbf{x}_i \mathbf{x}_i^{\mathrm{T}}\right)^{-1}\left(\tfrac{1}{n}\sum \mathbf{x}_i y_i\right).$$

Where X represents the matrix containing appropriate data from the explanatory variables and Y is the vector of dependent (or response) variables. This is essentially finding a least squares solution to fit parameters (i.e. levels of chemical specific product production) to the observed fed batch performance (here defined as Maximum MAb titer).

An example of a multiple linear model built from the plurality of clone microenvironments is

Maximum MAb. Titer = 37.78*(Control plate titer) - 22.4150*(MeIAB plate titer)

+ 0.4711

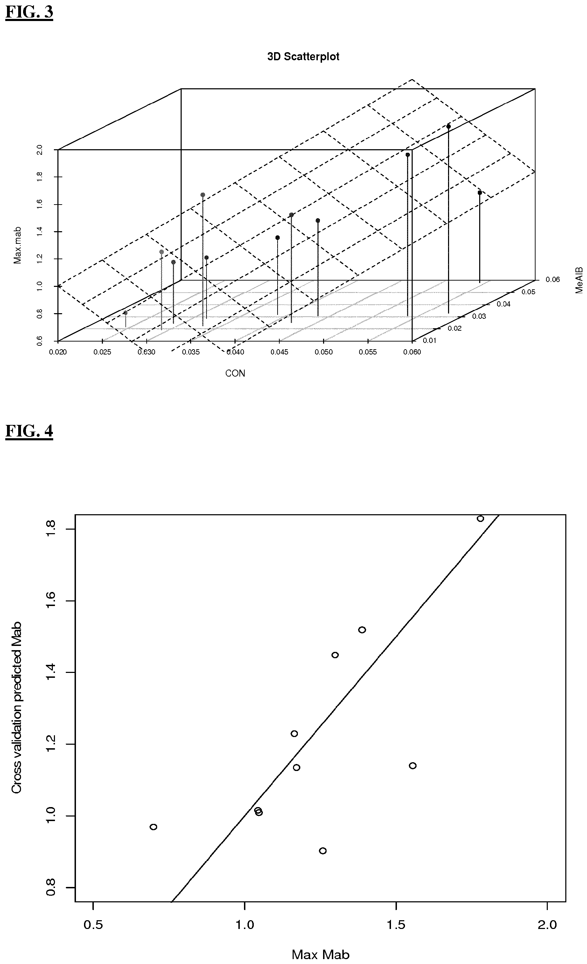

Where Maximum Mab. Titer is the maximum monoclonal antibody titer achieved in fed batch culture. A graph illustrating the goodness of model fit using the above parameters is shown in Fig. 3. The $R^2$ value for this model is 0.84. This can be interpreted that 84% of the behavior of the data can be explained by the model.

By using bootstrap cross validation techniques it can be shown that a model built in the above way can be predictive of future data, i.e. data not used in the model. Fig. 4 illustrates the predictive ability of the modeling approach to build predictive models. With this model, a multiple $R^2$ of 0.68 was achieved for predicting new data.

This model, build on a set of clones with known fed batch performance can use the 'fingerprint' of new clones and be able to predict their relative fed batch performance. Initially a model is generated using a panel of clones with known fed batch performance (in the above example 12 were used but there is no limitation to how many can be used - However the complexity of the model built is limited by the number of clones used). Subsequently when a large panel of clones is derived in the process of cell line development, these will be "screened" in the multiple microenvironment plates, and using the model generated from clones with known performance characteristics, it is possible to predict Maximum MAb Titer, rank the clones or at least give information as to the likelihood of their future fed batch performance capabilities therefore aiding the process of selecting which clones are taken forward to the next stage of clones screening (i.e. small scale fed batch studies).

The embodiments in the invention described optionally comprise a computer apparatus and/or processes performed in a computer apparatus. However, the invention also extends to computer programs, particularly computer programs stored on or in a carrier adapted to bring the invention into practice. The program may be in the form of source code, object code, or a code intermediate source and object code, such as in partially compiled form or in any other form suitable for use in the implementation of the method according to the invention. The carrier may comprise a storage medium such as ROM, e.g. CD ROM, or magnetic recording medium, e.g. a memory stick or hard disk. The carrier may be an electrical or optical signal which may be transmitted via an electrical or an optical cable or by radio or other means.

In the specification the terms "comprise, comprises, comprised and comprising" or any variation thereof and the terms include, includes, included and including" or any variation thereof are considered to be totally interchangeable and they should all be afforded the widest possible interpretation and vice versa.

## Claims

1. A rapid, high-throughput, computer-implemented method of predicting relative production titer of a panel of clonal producer cells in fed batch culture, the method comprising the steps of:

   - simultaneously incubating each producer cell with at least three individual chemical cell stressors in static microplate culture for an incubation period of less than four days, in which each producer cell is incubated with a single chemical cell stressor provided at a concentration of 0.5 to 2.0 $IC_{50}$;
   - after the incubation period determining the production titer responses of each cell in the presence and absence of the at least three chemical cell stressors to generate a cell-specific production titer response profile for each of the panel of producer cells;
- inputting the cell-specific production titer response profile for each of the panel of producer cells into a computational model, in which the computational model is generated from production titer response profiles obtained from a calibration set of producer cells with known fed batch production titre, wherein the computational model is configured to output the predicted relative fed batch production titer of the panel of producer cells.

2. A method according to Claim 1 in which the production titer responses comprise normalized production titer responses.

3. A method according to any preceding Claim, in which the production titer is the production titer of a recombinant protein, wherein the producer cell comprises a transgene encoding the recombinant protein; wherein the recombinant protein is optionally a monoclonal antibody, or a fragment thereof.

4. A method according to any preceding Claim, in which the panel of clonal producer cells are CHO cells.

5. A method according to any preceding Claim in which each producer cell is incubated with the single chemical cell stressor or each individual chemical cell stressor in wells of a microtitre plate in the static microplate culture.

6. A method according to any preceding Claim in which the single chemical cell stressor or each individual chemical cell stressor is employed at a concentration that is pre-determined to inhibit proliferation of the clonal producer cells during incubation within the LD30 to LD80 range.

7. A rapid, high-throughput, computer-implemented method of predicting relative production titer of a panel of clonal producer cells in fed batch culture according to any preceding Claim, the method comprising the steps of:

   - simultaneously incubating each producer cell with at least three individual, functionally diverse, chemical cell stressors in static microplate culture for an incubation period of less than four days in which each producer cell is incubated with a single chemical cell stressor provided at a concentration of 0.5 to 2.0 $IC_{50}$.;
   - after the incubation period determining the production titer response of each cell in the presence of each individual chemical cell stressors to generate a cell-specific production titer response profile for each of the panel of clonal producer cells;
   - inputting the cell-specific production titer response profile for each of the panel of producer cells into a computational model, in which the computational model is generated from production titer response profiles obtained from a calibration set of producer cells with known fed batch production titre, wherein the computational model is configured to output the predicted relative fed batch production titer of the panel of producer cells.

8. A computer implemented system for performing a method of predicting relative production titer of a panel of clonal producer cells in fed batch culture, the system comprising:

   - a determination system for assaying production titer of each clone in the presence and absence of at least three individual chemical cell stressors provided at a concentration of 0.5 to 2.0 $IC_{50}$ to provide a normalised production titer response value for each clone in a plurality of stressed microenvironments ;
   - a computational model adapted to process a clone-specific production titer response profile comprising the normalised production titer response value for each clone in each of the stressed microenvironments, in which the computational model is generated from production titer response profiles obtained from a calibration set of clones with known fed batch production titer response profiles, wherein the computational model is configured to output the predicted fed batch production titer value for each clone and/or the predicted relative fed batch production titer of the panel of clonal cells, and
   - means for outputting (i) the predicted fed batch production titre for one or more clones in the panel, or (ii) the predicted relative fed batch performance of the panel of clonal cells, or (iii) both (i) and (ii).

9. A system as claimed in Claim 8, in which the determination system comprises a HPLC.

10. A method according to any of Claims 1 to 7, or a system according to Claim 8 or 9, in which the computational model is a multiple linear computational model.

11. A method according to any of Claims 1 to 7, or a system according to Claims 8 to 9, in which the multiple linear computational model suitably employs a least squares solution to fit levels of cell specific production titer responses

to the observed fed batch production titer.

12. A method of screening a panel of producer cell clones derived from a single cell line to stratify the clones according to fed batch production titer, the method comprising steps of incubating a sample of each clone with and without at least three individual chemical cell stressors provided at a concentration of 0.5 to 2.0 $IC_{50}$ for a period of time of at least 24 hours and up to 4 or 5 days, to obtain a plurality of normalised production titer response values for the clone in which the production titer response value(s) provide a specific pattern of clone-specific production titer responses that forms a clone-specific production titer response profile, and employing a multiple linear computational model to correlate the clone-specific production titer response profiles with a plurality of production titer response profiles from a calibration set of clones with known fed batch production titer, and predict a fed batch production titer for one or more of the panel of clones.

13. A method according to Claim 12 in which the clones from the panel are ranked according to predicted fed batch production titer.

14. A computer program which when executed on a computer causes the computer to perform a method of predicting relative fed batch production titer of a panel of clonal cells derived from a single cell line according to the method of any of Claims 1 to 7 or 10 to 13.

15. A computer program recording medium storing a computer program according to Claim 14.

16. A computer-implemented method of predicting production titer of a query producer cell in fed batch culture, the method comprising the steps of:

   - incubating the query producer cell with at least three chemical cell stressors individually in static microplate culture, each chemical cell stressor provided at a concentration of 0.5 to 2.0 ICso;
   - determining the production titer response of the query producer cell in the presence of each chemical cell stressor to generate a query cell-specific production titer response profile;
   - inputting the query cell-specific production titer response profile into a computational model, in which the computational model is generated from production titer response profiles obtained from a calibration set of cells with known fed batch production titre, wherein the computational model is configured to output the predicted fed batch production titre for the cell.

**Patentansprüche**

1. Schnelles computerimplementiertes Hochdurchsatzverfahren zur Vorhersage von relativem Produktionstiter eines Panels von klonalen produzierenden Zellen in Fed-Batch-Kultur, wobei das Verfahren die folgenden Schritte umfasst:

   - gleichzeitiges Inkubieren von jeder produzierenden Zelle mit mindestens drei individuellen chemischen Zellstressoren in statischer Mikroplattenkultur für eine Inkubationsdauer von weniger als vier Tagen, bei dem jede produzierende Zelle mit einem einzelnen chemischen Zellstressor inkubiert wird, der in einer Konzentration von 0,5 bis 2,0 $IC_{50}$ bereitgestellt wird;
   - nach der Inkubationsdauer Bestimmen der Produktionstiter-Reaktionen von jeder Zelle in Gegenwart und Abwesenheit der mindestens drei chemischen Zellstressoren, um ein zellspezifisches Produktionstiter-Reaktionsprofil für jede des Panels von produzierenden Zellen zu erzeugen;
   - Eingeben des zellspezifischen Produktionstiter-Reaktionsprofils für jede des Panels von produzierenden Zellen in ein Berechnungsmodell, bei dem das Berechnungsmodell aus Produktionstiter-Reaktionsprofilen erzeugt wird, die aus einem Kalibriersatz von produzierenden Zellen mit bekanntem Fed-Batch-Produktionstiter erhalten werden, wobei das Berechnungsmodell zur Ausgabe des vorhergesagten relativen Fed-Batch-Produktionstiters des Panels von produzierenden Zellen konfiguriert ist.

2. Verfahren nach Anspruch 1, in dem die Produktionstiter-Reaktionen normalisierte Produktionstiter-Reaktionen umfassen.

3. Verfahren nach einem vorstehenden Anspruch, in dem der Produktionstiter der Produktionstiter eines rekombinanten Proteins ist, wobei die produzierende Zelle ein Transgen umfasst, das das rekombinante Protein kodiert; wobei das rekombinante Protein optional ein monoklonaler Antikörper oder ein Fragment davon ist.

**4.** Verfahren nach einem vorstehenden Anspruch, in dem das Panel von klonalen produzierenden Zellen CHO-Zellen ist.

**5.** Verfahren nach einem vorstehenden Anspruch, in dem jede produzierende Zelle mit dem einzelnen chemischen Zellstressor oder jedem individuellen chemischen Zellstressor in Wells einer Mikrotiterplatte in der statischen Mikroplattenkultur inkubiert wird.

**6.** Verfahren nach einem vorstehenden Anspruch, in dem der einzelne chemische Zellstressor oder jeder individuelle chemische Zellstressor in einer Konzentration eingesetzt wird, die vorgegeben ist, um Proliferation der klonalen produzierenden Zellen während der Inkubation innerhalb des LD30- bis LD80-Bereichs zu inhibieren.

**7.** Schnelles computerimplementiertes Hochdurchsatzverfahren zur Vorhersage von relativem Produktionstiter eines Panels von klonalen produzierenden Zellen in Fed-Batch-Kultur nach einem vorstehenden Anspruch, wobei das Verfahren die folgenden Schritte umfasst:

- gleichzeitiges Inkubieren von jeder produzierenden Zelle mit mindestens drei individuellen, funktionell verschiedenen, chemischen Zellstressoren in statischer Mikroplattenkultur für eine Inkubationsdauer von weniger als vier Tagen, bei dem jede produzierende Zelle mit einem einzelnen chemischen Zellstressor inkubiert wird, der in einer Konzentration von 0,5 bis 2,0 $IC_{50}$ bereitgestellt wird;
- nach der Inkubationsdauer Bestimmen der Produktionstiter-Reaktion von jeder Zelle in Gegenwart von jedem individuellen chemischen Zellstressor, um ein zellspezifisches Produktionstiter-Reaktionsprofil für jede des Panels von klonalen produzierenden Zellen zu erzeugen;
- Eingeben des zellspezifischen Produktionstiter-Reaktionsprofils für jede des Panels von produzierenden Zellen in ein Berechnungsmodell, bei dem das Berechnungsmodell aus Produktionstiter-Reaktionsprofilen erzeugt wird, die aus einem Kalibriersatz von produzierenden Zellen mit bekanntem Fed-Batch-Produktionstiter erhalten werden, wobei das Berechnungsmodell zur Ausgabe des vorhergesagten relativen Fed-Batch-Produktionstiters des Panels von produzierenden Zellen konfiguriert ist.

**8.** Computerimplementiertes System für die Durchführung eines Verfahrens zur Vorhersage von relativem Produktionstiter eines Panels von klonalen produzierenden Zellen in Fed-Batch-Kultur, wobei das System Folgendes umfasst:

- ein Bestimmungssystem für die Prüfung des Produktionstiters von jedem Klon in Gegenwart und Abwesenheit von mindestens drei individuellen chemischen Zellstressoren, die in einer Konzentration von 0,5 bis 2,0 $IC_{50}$ bereitgestellt werden, um einen normalisierten Produktionstiter-Reaktionswert für jeden Klon in einer Vielzahl von gestressten Mikroumgebungen bereitzustellen;
- ein Berechnungsmodell, das zur Verarbeitung eines klonspezifischen Produktionstiter-Reaktionsprofils geeignet ist, das den normalisierten Produktionstiter-Reaktionswert für jeden Klon in jeder der gestressten Mikroumgebungen umfasst, bei dem das Berechnungsmodell aus Produktionstiter-Reaktionsprofilen erzeugt wird, die aus einem Kalibriersatz von Klonen mit bekannten Fed-Batch-Produktionstiter-Reaktionsprofilen erhalten werden, wobei das Berechnungsmodell zur Ausgabe des vorhergesagten Fed-Batch-Produktionstiterwerts für jeden Klon und/oder des vorhergesagten relativen Fed-Batch-Produktionstiters des Panels von klonalen Zellen konfiguriert ist, und
- Mittel für die Ausgabe (i) des vorhergesagten Fed-Batch-Produktionstiters für einen oder mehrere Klone in dem Panel oder (ii) der vorhergesagten relativen Fed-Batch-Leistung des Panels von klonalen Zellen oder (iii) von sowohl (i) als auch (ii).

**9.** System nach Anspruch 8, in dem das Bestimmungssystem eine HPLC umfasst.

**10.** Verfahren nach einem der Ansprüche 1 bis 7 oder ein System nach Anspruch 8 oder 9, in dem das Berechnungsmodell ein multiples lineares Berechnungsmodell ist.

**11.** Verfahren nach einem der Ansprüche 1 bis 7 oder ein System nach den Ansprüchen 8 bis 9, in dem das multiple lineare Berechnungsmodell eine Lösung der kleinsten Quadrate passend einsetzt, um Höhen von zellspezifischen Produktionstiter-Reaktionen an den beobachteten Fed-Batch-Produktionstiter anzupassen.

**12.** Verfahren zum Screening eines Panels von produzierenden Zellklonen, die von einer einzelnen Zelllinie abstammen, um die Klone entsprechend dem Fed-Batch-Produktionstiter zu stratifizieren, wobei das Verfahren die folgenden Schritte umfasst: Inkubieren einer Probe von jedem Klon mit und ohne mindestens drei individuelle chemische Zellstressoren, die in einer Konzentration von 0,5 bis 2,0 $IC_{50}$ bereitgestellt werden, für eine Zeitdauer von mindestens 24 Stunden und bis zu 4 oder 5 Tage, um eine Vielzahl von normalisierten Produktionstiter-Reaktionswerten für den Klon zu erhalten, bei dem der/die Produktionstiter-Reaktionswert(e) ein spezifisches Muster von klonspezifischen Produktionstiter-Reaktionen bereitstellen, das ein klonspezifisches Produktionstiter-Reaktionsprofil bildet, und Einsetzen eines multiplen linearen Berechnungsmodell, um die klonspezifischen Produktionstiter-Reaktionsprofile mit einer Vielzahl von Produktionstiter-Reaktionsprofilen aus einem Kalibriersatz von Klonen mit bekanntem Fed-Batch-Produktionstiter zu korrelieren und einen Fed-Batch-Produktionstiter für einen oder mehrere des Panels von Klonen vorherzusagen.

13. Verfahren nach Anspruch 12, in dem die Klone von dem Panel entsprechend dem vorhergesagten Fed-Batch-Produktionstiter eingestuft werden.

14. Computerprogramm, das bei Ausführung an einem Computer bewirkt, dass der Computer ein Verfahren zur Vorhersage von relativem Fed-Batch-Produktionstiter eines Panels von klonalen Zellen, die von einer einzelnen Zelllinie abstammen, entsprechend dem Verfahren nach einem der Ansprüche 1 bis 7 oder 10 bis 13 ausführt.

15. Computerprogramm aufzeichnendes Medium, das ein Computerprogramm nach Anspruch 14 speichert.

16. Computerimplementiertes Verfahren zur Vorhersage von Produktionstiter einer produzierenden Query-Zelle in Fed-Batch-Kultur, wobei das Verfahren die folgenden Schritte umfasst:

   - Inkubieren der produzierenden Query-Zelle mit mindestens drei chemischen Zellstressoren individuell in statischer Mikroplattenkultur, wobei jeder chemische Zellstressor in einer Konzentration von 0,5 bis 2,0 $IC_{50}$ bereitgestellt wird;
   - Bestimmen der Produktionstiter-Reaktion der produzierenden Query-Zelle in Gegenwart von jedem chemischen Zellstressor, um ein zellspezifisches Query-Produktionstiter-Reaktionsprofil zu erzeugen;
   - Eingeben des zellspezifischen Query-Produktionstiter-Reaktionsprofils in ein Berechnungsmodell, bei dem das Berechnungsmodell aus Produktionstiter-Reaktionsprofilen erzeugt wird, die aus einem Kalibriersatz von Zellen mit bekanntem Fed-Batch-Produktionstiter erhalten werden, wobei das Berechnungsmodell zur Ausgabe des vorhergesagten Fed-Batch-Produktionstiters für die Zelle konfiguriert ist.

## Revendications

1. Procédé rapide et à haut débit mis en oeuvre par informatique de prédiction de titre de production relatif d'un panel de cellules productrices clonales en culture en alimentation programmée, le procédé comprenant les étapes consistant à :

   - incuber simultanément chaque cellule productrice avec au moins trois facteurs de stress cellulaire chimiques individuels en culture en microplaque statique pendant une période d'incubation inférieure à quatre jours, au cours de laquelle chaque cellule productrice est incubée avec un unique facteur de stress cellulaire chimique fourni à une concentration de 0,5 à 2,0 CIso ;
   - après la période d'incubation, déterminer les réponses de titre de production de chaque cellule en la présence et en l'absence desdits au moins trois facteurs de stress cellulaire chimique pour générer un profil de réponse de titre de production propre à la cellule pour chaque panel de cellules productrices ;
   - entrer le profil de réponse de titre de production propre à la cellule pour chaque panel de cellules productrices dans un modèle de calcul, le modèle de calcul étant généré à partir des profils de réponse de titre de production obtenus à partir d'une série d'étalonnage de cellules productrices de titre de production en alimentation programmée connu, le modèle de calcul étant configuré pour retourner le titre de production en alimentation programmée relatif prévu du panel de cellules productrices.

2. Procédé selon la revendication 1, les réponses de titre de production comprenant des réponses de titre de production normalisées.

3. Procédé selon l'une quelconque des revendications précédentes, le titre de production étant le titre de production d'une protéine recombinante, la cellule productrice comprenant un transgène codant pour la protéine recombinante ; la protéine recombinante étant de manière facultative un anticorps monoclonal, ou un fragment de celui-ci.

**4.** Procédé selon l'une quelconque des revendications précédentes, le panel de cellules productrices clonales étant des cellules ovariennes de hamster chinois.

**5.** Procédé selon l'une quelconque des revendications précédentes, chaque cellule productrice étant incubée avec l'unique facteur de stress cellulaire chimique ou chaque facteur de stress cellulaire chimique individuel dans des puits d'une plaque de microtitrage de la culture en microplaque statique.

**6.** Procédé selon l'une quelconque des revendications précédentes, l'unique facteur de stress cellulaire chimique ou chaque facteur de stress chimique cellulaire individuel étant employé à une concentration qui est prédéterminée de sorte à inhiber une prolifération des cellules productrices clonales durant l'incubation au sein de la plage DL30 à DL80.

**7.** Procédé rapide et à haut débit mis en oeuvre par informatique de prédiction de titre de production relatif d'un panel de cellules productrices clonales en culture en alimentation programmée selon l'une quelconque des revendications précédentes, le procédé comprenant les étapes consistant à :

- incuber simultanément chaque cellule productrice avec au moins trois facteurs de stress cellulaire chimiques individuels, fonctionnellement variés, en culture en microplaque statique pendant une période d'incubation inférieure à quatre jours, au cours de laquelle chaque cellule productrice est incubée avec un unique facteur de stress cellulaire chimique fourni à une concentration de 0,5 à 2,0 $CI_{50}$ ;
- après la période d'incubation, déterminer la réponse de titre de production de chaque cellule en la présence de chaque facteur de stress cellulaire chimique individuel pour générer un profil de réponse de titre de production propre à chaque cellule pour chaque panel de cellules productrices clonales ;
- entrer le profil de réponse de titre de production propre à la cellule pour chaque panel de cellules productrices clonales dans un modèle de calcul, le modèle de calcul étant généré à partir des profils de réponse de titre de production obtenus à partir d'une série d'étalonnage de cellules productrices de titre de production en alimentation programmée connu, le modèle de calcul étant configuré pour retourner le titre de production en alimentation programmée relatif prévu du panel de cellules productrices.

**8.** Système mis en oeuvre par informatique destiné à effectuer un procédé de prédiction de titre de production relatif d'un panel de cellules productrices clonales en culture en alimentation programmée, le système comprenant :

- un système de détermination destiné à doser un titre de production de chaque clone en la présence et en l'absence d'au moins trois facteurs de stress cellulaire chimique individuels fournis à une concentration de 0,5 à 2,0 $CI_{50}$ pour fournir une valeur de réponse de titre de production normalisée pour chaque clone dans une pluralité de micro-environnements stressés ;
- un modèle de calcul approprié au traitement d'un profil de réponse de titre de production propre au clone comprenant la valeur de réponse de titre de production normalisée pour chaque clone dans chacun des micro-environnements stressés, le modèle de calcul étant généré à partir de profils de réponse de titre de production obtenus à partir d'une série d'étalonnage de clones de profils de réponse de titre de production en alimentation programmée connus, le modèle de calcul étant configuré pour retourner la valeur de titre de production en alimentation programmée prédite pour chaque clone et/ou le titre de production en alimentation programmée relatif prédit du panel de cellules clonales, et
- un moyen d'émission (i) du titre de production en alimentation programmée prédit pour un ou plusieurs clones du panel, ou (ii) de la performance en alimentation programmée relative prédite du panel de cellules clonales, ou (iii) de (i) et de (ii) à la fois.

**9.** Système selon la revendication 8, le système de détermination comprenant une HPLC.

**10.** Procédé selon l'une quelconque des revendications 1 à 7, ou système selon la revendication 8 ou 9, le modèle de calcul étant un modèle de calcul linéaire multiple.

**11.** Procédé selon l'une quelconque des revendications 1 à 7, ou système selon les revendications 8 à 9, le modèle de calcul linéaire multiple utilisant de manière appropriée une résolution des moindres carrés pour ajuster les niveaux des réponses de titre de production propres aux cellules au titre de production en alimentation programmée observé.

**12.** Procédé de criblage d'un panel de clones de cellules productrices dérivées d'une unique lignée cellulaire pour stratifier les clones en fonction d'un titre de production en alimentation programmée, le procédé comprenant les étapes consistant à incuber un échantillon de chaque clone avec et sans au moins trois facteurs de stress cellulaire chimique individuels fournis à une concentration de 0,5 à 2,0 $CI_{50}$ pendant une période d'au moins 24 heures et jusqu'à 4 ou 5 jours, pour obtenir une pluralité de valeurs de réponse de titre de production normalisées pour le clone, la ou les valeurs de réponse de titre de production fournissant un motif spécifique des réponses de titre de production propre au clone qui forme un profil de réponse de titre de production propre au clone, et utiliser un modèle de calcul linéaire multiple pour corréler les profils de réponse de titre de production propre au clone avec une pluralité de profils de réponse de titre de production d'une série d'étalonnage de clones de titres de production en alimentation programmée connus, et prédire un titre de production en alimentation programmée pour un ou plusieurs du panel de clones.

13. Procédé selon la revendication 12, les clones du panel étant classés en fonction du titre de production en alimentation programmée prédit.

14. Programme informatique qui lorsqu'il est exécuté sur un ordinateur amène l'ordinateur à effectuer un procédé de prédiction de titre de production en alimentation programmée relatif d'un panel de cellules clonales dérivées d'une unique lignée cellulaire selon le procédé de l'une quelconque des revendications 1 à 7 ou 10 à 13.

15. Support d'enregistrement de programme informatique mémorisant un programme informatique selon la revendication 14.

16. Procédé mis en oeuvre par informatique de prédiction de titre de production d'une cellule productrice demandée en culture en alimentation programmée, le procédé comprenant les étapes consistant à :

    - incuber la cellule productrice demandée avec au moins trois facteurs de stress chimique individuellement en culture en microplaque statique, chaque facteur de stress chimique étant fourni à une concentration de 0,5 à 2,0 $CI_{50}$ ;
    - déterminer la réponse de titre de production de la cellule productrice demandée en la présence de chaque facteur de stress cellulaire chimique pour générer un profil de réponse de titre de production propre à la cellule demandée ;
    - entrer le profil de réponse de titre de production propre à la cellule demandée dans un modèle de calcul, le modèle de calcul étant généré à partir des profils de réponse de titre de production obtenus à partir d'une série d'étalonnage de cellules de titre de production en alimentation programmée connus, le modèle de calcul étant configuré pour retourner le titre de production en alimentation programmée prédit pour la cellule.

**FIG. 1**

**FIG. 2**

## FIG. 3

**3D Scatterplot**

## FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WURM ; FLORIAN M.** Production of recombinant protein therapeutics in cultivated mammalian cells. *Nature Biotechnology,* 2004, vol. 22, 1393-1398